# EUROPÄISCHE PATENTANMELDUNG

(11) **EP 3 991 679 A1**
(43) Veröffentlichungstag der Anmeldung: **04.05.2022**
(21) Anmeldenummer: 21199390.2
(22) Anmeldetag: 28.09.2021
(51) Int. Cl.: A61B 18/14, A61B 18/12, A61B 90/00

(54) **ELEKTROCHIRURGISCHES INSTRUMENT UND ELEKTROCHIRURGIESYSTEM MIT EINEM ELEKTROCHIRURGIE-GENERATOR**

(30) Priorität: 30.10.2020 DE 102020128692
(71) Anmelder: Olympus Winter & Ibe GmbH, 22045 Hamburg (DE)
(72) Erfinder: Janich, Fabian, 14469 Potsdam (DE); Kwik, Anne, 14165 Berlin (DE); Breitsprecher, Frank, 12527 Berlin (DE); Krüger, Jens, 15732 Eichwalde (DE)
(74) Vertreter: Eisenführ Speiser

(57) **Zusammenfassung**

Die Erfindung betrifft ein elektrochirurgisches Instrument (14) mit wenigstens einer aktiven Elektrode (22) zum Bewirken einer elektrochirurgischen Behandlung und einem Instrumenten-Datenspeicher (26). Außerdem weist das elektrochirurgische Instrument einen Anschluss für ein Anschlusskabel oder ein angeschlossenes Anschlusskabel (16) auf, das Versorgungsleitungen zum Versorgen der aktiven Elektrode mit einem für eine elektrochirurgische Behandlung benötigten hochfrequenten Wechselstrom umfasst. Der Instrumenten-Datenspeicher (26) weist zumindest eine Schreib-Lese-Bereich (Read-Write-Area) auf, der von einem Elektrochirurgie-Generator (12) mit Nutzungs- und Zustandsdaten beschrieben werden kann.

## Beschreibung

Die Erfindung betrifft ein elektrochirurgisches Instrument und ein Elektrochirurgiesystem mit einem Elektrochirurgie-Generator, um das elektrochirurgische Instrument mit hochfrequentem Wechselstrom zu versorgen.

Ein Elektrochirurgiesystem umfasst in der Regel einen Elektrochirurgie-Generator zum Erzeugen des hochfrequenten Wechselstroms. Der Elektrochirurgie-Generator hat in der Regel zwei oder mehr Ausgänge, an die ein elektrochirurgisches Instrument angeschlossen werden kann und zwischen denen im Betrieb eine hochfrequente Wechselspannung bereitgestellt wird. Dazu umfasst ein Elektrochirurgie-Generator in der Regel ein Hochspannungs-Netzteil, das im Betrieb einen Gleichstrom erzeugt, und einen Hochfrequenz-Teil, der mit dem Hochspannungs-Netzteil verbunden ist und im Betrieb aus dem Gleichstrom einen hochfrequenten Wechselstrom erzeugt.

An den Elektrochirurgie-Generator können typischerweise verschiedene elektrochirurgische Instrumente für unterschiedliche Aufgaben angeschlossen werden, die unterschiedliche und von dem jeweiligen elektrochirurgischen Instrument abhängige Anforderungen an den Generator stellen.

Mittels Elektrochirurgie kann biologisches Gewebe - also Körpergewebe - geschnitten, koaguliert (verödet) und/oder vaporisiert werden. Für die Elektrochirurgie werden typischerweise hochfrequente Wechselströme mit einer Frequenz zwischen 0,2 MHz und 3 MHz verwendet. Elektrochirurgische Instrumente sind typischerweise Handinstrumente, mit denen ein Arzt Körpergewebe koagulieren, ablatieren und/oder schneiden kann.

Elektrochirurgische Instrumente werden hierzu mit hochfrequenter elektrischer Energie gespeist, mit der Gewebe gezielt koaguliert oder geschnitten werden kann. Die hochfrequente elektrische Energie wird von dem Elektrochirurgie-Generator bereitgestellt und mittels des jeweils geeigneten elektrochirurgischen Instruments auf Körpergewebe angewandt. Je nach Anwendung sind spezielle Strom- und Spannungsverläufe notwendig, die dem Arzt in Form von Betriebsmodi (auch Modes genannt) zur Auswahl am Elektrochirurgie-Generator zur Verfügung gestellt werden. Diese Betriebsmodi sind fest im Elektrochirurgie-Generator hinterlegt.

Aus WO 2011/032729 A1 ist eine einfach programmierbare Versorgungseinrichtung für ein elektrochirurgisches Instrument bekannt. Die einfache Programmierbarkeit resultiert aus definiert vorgegeben möglichen Zuständen eines Zustandsautomaten, die allerdings auch die Flexibilität der Versorgungseinrichtung einschränken.

Der Erfindung liegt die Aufgabe zugrunde, ein elektrochirurgisches Instrument zu schaffen, das mit einem Elektrochirurgiesystem flexibel und sicher betrieben werden kann.

Erfindungsgemäß werden hierzu ein elektrochirurgisches Instrument, ein Elektrochirurgiesystem und ein Elektrochirurgie-Generator vorgeschlagen, die jeweils für sich und in Kombination miteinander ein einerseits sehr flexibles und andererseits auf einen jeweiligen Anwendungsfall bzw. für ein jeweiliges elektrochirurgisches Instrument maßgeschneiderte Betriebsmodi ermöglichen.

Ein erfindungsgemäßes elektrochirurgisches Instrument umfasst wenigstens eine aktive Elektrode zum Bewirken einer elektrochirurgischen Behandlung und einen Instrumenten-Datenspeicher. Außerdem weist das elektrochirurgische Instrument einen Anschluss für ein Anschlusskabel oder ein angeschlossenes Anschlusskabel auf, das Versorgungsleitungen zum Versorgen der aktiven Elektrode mit einem für eine elektrochirurgische Behandlung benötigten hochfrequenten Wechselstrom umfasst. Weiterhin umfasst das elektrochirurgische Instrument wenigstens eine Datenschnittstelle zum Auslesen von Daten aus dem Datenspeicher sowie zum Schreiben von Daten in den Datenspeicher. Die Datenschnittstelle kann für einen drahtgebundenen oder einen drahtlosen Datenaustausch zwischen einem Elektrochirurgie-Generator und dem elektrochirurgischen Instrument konfiguriert sein. Der Instrumenten-Datenspeicher ist vorzugsweise partitioniert und weist vorzugsweise zumindest eine Schreib-Lese-Partition (Read-Write-Area) und einen Konfigurationsbereich (Config Area) auf, von denen die Schreib-Lese-Partition mit Nutzungs- und Zustandsdaten beschrieben werden kann.

Das Beschreiben der Schreib-Lese-Partition mit Nutzungs- und Zustandsdaten kann im Betrieb des elektrochirurgischen Instruments durch einen angeschlossenen Elektrochirurgie-Generator erfolgen. Damit ist es möglich, einen sicheren Betrieb des elektrochirurgischen Instruments mit einem Elektrochirurgie-Generator zu gewährleisten. Nutzungs- und Zustandsdaten können z. B. Aktivierungs- und Steckzeiten, Aktivierungsenergien, Fehlerzustände oder verbleibende Aktivierungszyklen des elektrochirurgischen Instruments sein. Die Definition der zu speichernden Nutzungs- und Zustandsdaten kann vollständig in einem Nur-Lese-Bereich (Read-Only-Area) des Instrumenten-Datenspeichers hinterlegt werden, so dass auch nach Markteinführung eines neuen Elektrochirurgie-Generators Anpassungen der Nutzungs- und Zustandsdaten über die Programmierung des elektrochirurgischen Instrumentes möglich sind.

Vorzugsweise enthält der Instrumenten-Datenspeicher des elektrochirurgischen Instruments Datensätze mit strukturierten Daten, die Verweise auf in einem Generator-Datenspeicher eines Elektrochirurgie-Generators gespeicherte Betriebsvorgaben enthalten. Ein derartiges elektrochirurgisches Instrument kann somit die nötigen Informationen enthalten, die eine auf das jeweilige elektrochirurgische Instrument abgestimmten Betriebsmodus erlauben, ohne dass dieser vollständig durch das elektrochirurgische Instrument definiert zu sein braucht.

Für die Datensätze mit strukturierten Daten, die Verweise auf in einem Generator-Datenspeicher eines Elektrochirurgie-Generators gespeicherte Betriebsvorgaben enthalten, weist der Instrumenten-Datenspeicher des elektrochirurgischen Instruments vorzugsweise eine weitere Partition auf, die im Betrieb des elektrochirurgischen Instruments nur ausgelesen, nicht aber beschrieben werden kann. Entsprechend weist der Instrumenten-Datenspeicher vorzugsweise einen Nur-Lese-Bereich (Read-only Area) als weitere Partition auf. Die in dem Nur-Lese-Bereich des elektrochirurgischen Instruments gespeicherten Datensätze mit strukturierten Daten sind passend zu einer Datenstruktur in einem Generator-Datenspeicher eines Elektrochirurgie-Generators, mit dem das elektrochirurgische Instrument betrieben werden kann und die von einer Vielzahl von Datensätzen mit strukturierten Daten gebildet sein kann. Die strukturierten Daten enthalten Verweise auf konkrete Betriebsvorgaben aus einer Vielzahl von Betriebsvorgaben, die in dem Generator-Datenspeicher des Elektrochirurgie-Generators gespeichert sind, und definieren diejenigen konkreten Betriebsvorgaben wie Steuerbefehle oder Parameterwerte, die für den jeweiligen Betrieb des elektrochirurgischen Instruments in einem bestimmten Betriebsmodus zur Anwendung kommen sollen. Die durch die strukturierten Daten definierten Verweise werden im Betrieb des Elektrochirurgie-Generators von einem Prozessor gemäß eines dann ablaufenden Betriebsprogramms aufgerufen und in Betriebsvorgaben übersetzt, die der Prozessor in Verbindung mit dem Betriebsprogramm dann anwenden kann. Dies ist nachfolgend im Zusammenhang mit der Beschreibung des Elektrochirurgie-Generators noch näher erläutert.

Das elektrochirurgische Instrument ist vorzugsweise ein Handinstrument mit einem Handgriff und einem Schaft, an dem die aktive Elektrode angeordnet ist. Der Datenspeicher ist vorzugsweise in dem Handgriff des elektrochirurgischen Instruments untergebracht.

Der Instrumenten-Datenspeicher des elektrochirurgischen Instruments ist vorzugsweise nicht-flüchtig, aberwiederbeschreibbar. Dies ist deshalb vorteilhaft, weil ein solcher Datenspeicher mit aktuellen Zustands- und Nutzungsdaten zu dem elektrochirurgischen Instrument beschrieben werden kann und diese Zustands- und Nutzungsdaten auch bei Nichtgebrauch des elektrochirurgischen Instruments erhalten bleiben.

Vorzugsweise ist wenigstens die Struktur der den Schreib-Lese-Bereichs des Instrumenten-Datenspeichers durch eine Datenallokationstabelle definiert, die vorzugsweise auf einem Elektrochirurgie-Generator zum Betreiben des elektrochirurgischen Instruments gespeichert ist.

Vorzugsweise ist das elektrochirurgische Instrument Teil eines Elektrochirurgiesystems, das neben dem elektrochirurgischen Instrument auch einen Elektrochirurgie-Generator umfasst, an den das elektrochirurgische Instrument im Betrieb angeschlossen ist. Der Elektrochirurgie-Generator verfügt über einen Prozessor und mindestens einen Generator-Datenspeicher, in dem ein Betriebsprogramm zur Steuerung des Betriebs des Elektrochirurgie-Generators in Verbindung mit dem elektrochirurgischen Instrument gespeichert ist. In dem Generator-Datenspeicher des Elektrochirurgie-Generators sind unabhängig vom Anschluss eines elektrochirurgischen Instruments eine theoretisch beliebige Anzahl von Betriebsvorgaben gespeichert, die von dem Betriebsprogramm aufgerufen werden und somit potentiell zur Anwendung kommen können und den Betrieb des Elektrochirurgie-Generator beeinflussen können, aber keine festen Betriebsabläufe vorgeben. Die Betriebsvorgaben können einerseits Steuerbefehle, Befehlsausdrücke und/oder bedingte Ausdrücke usw. sein, andererseits aber auch parametrische Daten wie zum Beispiel Werte für die Ausgangsspannung, Ausgangsströme, Ausgangsleistung, Zeiteinstellungen, Schwellwerte, Fehlerbedingungen usw.

Die in dem Generator-Datenspeicher gespeicherten Betriebsvorgabenkönnen als Bibliothek einer beliebigen Vielzahl von potentiell anwendbaren Betriebsvorgaben verstanden werden, auf die das Betriebsprogramm zugreifen kann. Allerdings erfolgt der Zugriff nicht unmittelbar, so dass die Betriebsweise des Elektrochirurgie-Generator durch das Betriebsprogramm und die Betriebsvorgaben als Inhalte des Generator-Datenspeichers noch nicht bestimmt ist.

Vielmehr ist zusätzlich eine Datenstruktur vorgesehen, in der beliebige Datensätze mit strukturierten Daten gespeichert sind. Die strukturierten Daten enthalten Verweise auf konkrete Betriebsvorgaben wie Steuerbefehle oder Parameterwerte, die in dem Generator-Datenspeicher gespeichert sind. Die durch die strukturierten Daten definierten Verweise werden im Betrieb des Elektrochirurgie-Generators von dem Prozessor gemäß dem dann ablaufenden Betriebsprogramm aufgerufen und die zugehörigen Betriebsvorgaben werden von dem Betriebsprogramm angewandt.

Die konkrete Betriebsweise des Elektrochirurgie-Generators hängt somit von drei verschiedenen Arten von gespeicherten Daten ab, nämlich
- von den das Betriebsprogramm definierenden Daten in dem Generator-Datenspeicher,
- von den potentiell anzuwendenden Betriebsvorgaben in dem Generator-Datenspeicher, die beispielsweise Steuerbefehle oder Parameterwerte definieren, und von denen einige aufgrund von in der Datenstruktur enthaltenen Verweise konkret zur Anwendung kommen
- von den in der Datenstruktur gespeicherten Datensätzen mit strukturierten Daten, die die Verweise auf konkret anzuwendende Betriebsvorgaben enthalten und die während des Ablaufs des Betriebsprogramms von dem Prozessor aufgerufen und in konkret anzuwendende Betriebsvorgaben übersetzt werden.

Die drei Arten von Daten - Betriebsprogramm, Betriebsvorgaben und strukturierte Daten der Datenstruktur - können unabhängig geändert und vorgegeben werden, mit der Einschränkung, dass die Datenformate kompatibel sind. Die Betriebsweise des Elektrochirurgie-Generators in einem jeweiligen Betriebsmodus kann somit durch Ändern des in dem Generator-Datenspeicher gespeicherten Betriebsprogramm geändert werden, oder durch Ändern der ebenfalls in dem Generator-Datenspeicher gespeicherten Betriebsvorgaben oder durch Ändern der strukturierten Daten in der Datenstruktur oder auch durch eine Kombination dieser Änderungen. Die Datenstruktur kann entweder in dem Generator-Datenspeicher oder dem Instrumenten-Datenspeicher oder in beiden Datenspeichern vorliegen.

Der Generator-Datenspeicher enthält vorzugsweise eine Datenallokationstabelle, die wenigstens die Struktur des Schreib-Lese-Bereichs des Instrumenten-Datenspeichers des elektrochirurgischen Instruments definiert.

Das elektrochirurgische Instrument enthält den vorzugsweise nichtflüchtigen Instrumenten-Datenspeicher (z.B. ein EEPROM oder ähnliches), aber keinen Prozessor. Auf dem Instrumenten-Datenspeicher des chirurgischen Instruments befindet sich neben den im Schreib-Lese-Bereich gespeicherten Nutzungs- und Zustandsdaten ein vorzugsweise in dem Nur-Lese-Bereich gespeicherter Datensatz mit strukturierten Daten, die Verweise auf Betriebsvorgaben und ggf. Parameterwerte enthalten. Die strukturierten Daten dieses Datensatzes sind zu der Datenstruktur des Elektrochirurgie-Generators kompatibel und können die Datenstruktur des Elektrochirurgie-Generators oder einen Teil der Datenstruktur des Elektrochirurgie-Generators bilden.

Die Datenstruktur kann somit Teil des Generator-Datenspeichers sein, der physischer Bestandteil des Elektrochirurgie-Generators ist, oder sie wird durch den Inhalt des Instrumenten-Datenspeichers des elektrochirurgischen Instruments gebildet oder von einer Kombination aus einem Generator-Datenspeicher des Elektrochirurgie-Generators und dem Instrumenten-Datenspeicher des elektrochirurgischen Instruments. Vorzugsweise ist die Datenstruktur wenigstens zum Teil in einer eigenen Partition des Datenspeichers des elektrochirurgischen Instruments, nämlich dem Nur-Lese-Bereich, gespeichert.

Der Prozessor des Elektrochirurgie-Generators ist in Verbindung mit dem Betriebsprogramm dazu konfiguriert, den Instrumenten-Datenspeicher des elektrochirurgischen Instruments auszulesen und zu beschreiben. Die vorzugsweise vorgesehene Partitionierung des Speichers des elektrochirurgischen Instruments stellt hierbei sicher, dass die Datenstruktur in der Partition, die den Nur-Lese-bereich bildet, von dem Elektrochirurgie-Generator in dem normalen elektrochirurgischen Betrieb nur ausgelesen, nicht aber beschrieben werden kann. Die Partition mit dem Schreib-Lese-Bereich hingegen kann - und soll - von dem Elektrochirurgie-Generator beschrieben werden können, und zwar mit Zustands-und Nutzungsdaten zu dem jeweiligen elektrochirurgischen Instrument. Hierzu ist in dem Generator-Datenspeicher eine Datenallokationstabelle hinterlegt.

Das Schreiben von Zustands-und Nutzungsdaten erfolgt vorzugsweise durch Definition von entsprechenden Verweisen auf Steuerbefehle innerhalb der strukturierten Daten. Gespeichert werden die Daten dann entsprechend der Datenallokationstabelle, die im Generator definiert ist (Tag1 + Wert1, Tag2 + Wert2, ...).

Das Lesen von Zustands- und Nutzungsdaten erfolgt ebenfalls über die Datenallokationstabelle. Der Elektrochirurgie-Generator liest z. B. nach dem Anschließen eines elektrochirurgischen Instruments an den Elektrochirurgie-Generator die Schreib-Lese-Partition des Instrumenten-Datenspeichers aus und dekodiert die Zustands- und Nutzungsdaten entsprechend der Datenallokationstabelle und kann selbstständig damit agieren, um z. B. Instrumenteninformation anzuzeigen.

Der Prozessor des Elektrochirurgie-Generators kann durch das Betriebsprogramm dazu konfiguriert sein, den Instrumenten-Datenspeicher des elektrochirurgischen Instruments - insbesondere den Nur-Lese-Bereich und den Schreib-Lese-Bereich -, auszulesen, nachdem das elektrochirurgische Instrument an den Elektrochirurgie-Generator angeschlossen wurde und bevor der Elektrochirurgie-Generator in einem Betriebsmodus betrieben wird. Der Prozessor des Elektrochirurgie-Generators kann durch das Betriebsprogramm dazu konfiguriert sein, den Datensatz oder die Datensätze oder die in dem Datensatz oder den Datensätzen enthaltenen strukturierten Daten in eine Datenstruktur zu übertragen, die in dem Generator-Datenspeicher des Elektrochirurgie-Generators hinterlegt ist.

Der Prozessor des Elektrochirurgie-Generators kann durch das Betriebsprogramm alternativ dazu konfiguriert sein, den Instrumenten-Datenspeicher des elektrochirurgischen Instruments während des Ablaufs des Betriebsprogramms - also während der Elektrochirurgie-Generator in einem Betriebsmodus betrieben wird - auszulesen. In dem Instrumenten-Datenspeicher des chirurgischen Instruments ist somit kein Computerprogramm oder Algorithmus gespeichert, und die strukturierten Daten sind weder als Computerprogramm noch als ein mit einem Programm verbundener Algorithmus lesbar.

Die strukturierten Daten - auch die in der Nur-Lese-Bereich bildenden Partition des Datenspeichers des elektrochirurgischen Instruments gespeicherten strukturierten Daten -enthalten vorzugsweise parametrisierte Verweise. Die Verweise erlauben den gezielten Aufruf von Betriebsvorgaben wie z.B. Steuerbefehlen mittels des Betriebsprogramms.

Die strukturierten Daten liegen vorzugsweise in einem speichereffizienten Binärformat vor, bestehend aus einem 1-Byte-großen Verweis und für die Betriebsvorgabe zusätzlich erforderliche Informationen.

Vorzugsweise weist das Elektrochirurgiesystem eine Programmierschnittstelle oder mehrere Programmierschnittstellen auf, mittels der die Inhalte insbesondere des Generator-Datenspeichers und insbesondere der Datenstruktur mit den strukturierten Daten programmiert werden können. Entsprechend kann auch vorgesehen sein, dass das Betriebsprogramm in dem Generator-Datenspeicher über eine Programmierschnittstelle geändert werden kann, beispielsweise auf dem Wege eines Softwareupdates des Elektrochirurgie-Generators via USB.

Erfindungsgemäß wird auch ein Verfahren zum Betreiben eines erfindungsgemäßen elektrochirurgischen Instruments mittels eines Elektrochirurgie-Generators vorgeschlagen, demgemäß der Elektrochirurgie-Generator im Betrieb des elektrochirurgischen Instruments Nutzungs- und Zustandsdaten zu dem elektrochirurgischen Instrument in die den Schreib-Lese-Bereich (Read-Write-Area) des Datenspeichers schreibt.

Vorzugsweise sind Betriebsvorgaben und eine Datenstruktur in voneinander unabhängigen Speicherbereichen des Generator-Datenspeichers des Elektrochirurgie-Generators bereitgestellt, wobei die Datenstruktur Verweise auf die Betriebsvorgaben enthält und der Prozessor während des Ablaufs eines Betriebsprogramms indirekt auf einzelne Betriebsvorgaben zugreift, indem zunächst ein Zugriff auf Verweise in der Datenstruktur erfolgt und daraufhin ein Abruf derjenigen Betriebsvorgabe oder Betriebsvorgaben erfolgt, auf die ein jeweiliger Verweis verweist.

Vorzugsweise wird die Datenstruktur aus dem Instrumenten-Datenspeicher des elektrochirurgischen Instruments ausgelesen, und zwar vorzugsweise nach Anschluss und vor einer Anwendung eines elektrochirurgischen Instruments. Die in der Datenstruktur auf dem elektrochirurgischen Instrument enthaltenen Datensätze mit strukturierten Daten können in eine Datenstruktur übertragen werden, die auf einem Elektrochirurgie-Generator gespeichert ist.

Ein weiterer Aspekt der Erfindung ist ein Elektrochirurgie-Generator für den Betrieb des elektrochirurgischen Instruments. Der Elektrochirurgie-Generator besitzt Anschlüsse zum Anschließen des elektrochirurgischen Instruments sowie einen Prozessor und mindestens einen Generator-Datenspeicher, der
- erste Daten enthält, die ein Betriebsprogramm zur Steuerung des Betriebs des Elektrochirurgie-Generators in Verbindung mit dem elektrochirurgischen Instrument definieren,
- zweite Daten enthält, die Betriebsvorgaben definieren, die von dem Betriebsprogramm aufgerufen werden können und den Betrieb des Elektrochirurgie-Generator beeinflussen können, aber keine festen Betriebsabläufe vorgeben, und
- dritte Daten enthält, die eine Datenstruktur definieren, die Datensätze mit strukturierten Daten enthält, die Verweise auf einzelne Betriebsvorgaben enthalten.

Der Elektrochirurgie-Generator ist ausgebildet, beim Anschließen eines erfindungsgemäßen elektrochirurgischen Instrumentes an den Elektrochirurgie-Generator in dem Datenspeicher des elektrochirurgischen Instruments gespeicherte Nutzungs- und Zustandsdaten auszulesen sowie im Betrieb mit einem erfindungsgemäßen elektrochirurgischen Instrument Nutzungs- und Zustandsdaten in einem Schreib-Lese-Bereich des Instrumenten-Datenspeichers des elektrochirurgischen Instruments zu speichern.

Ein derartiger Elektrochirurgie-Generator kann leicht mit verschiedenen unterschiedlichen elektrochirurgischen Instrumenten verwendet werden, wobei die Anpassung ein jeweiliges elektrochirurgisches Instrument allein durch entsprechende, Verweise repräsentierende Einträge in der Datenstruktur erfolgen kann, ohne dass die Betriebsvorgaben in dem Generator-Datenspeicher des Elektrochirurgie-Generators geändert werden müssen. Insbesondere ist es möglich, den Betreib eines konkreten elektrochirurgischen Instruments auch von auf diesem elektrochirurgischen Instrument hinterlegten Zustands- und Nutzungsdaten abhängig zu machen.

Die Erfindung soll nun anhand von Ausführungsbeispielen mit Bezug auf die Figuren näher erläutert werden. Von den Figuren zeigt:
- Figur 1:: ein Elektrochirurgiesystem mit einem Elektrochirurgie-Generator und einem an diesem angeschlossenen elektrochirurgischen Instrument;
- Figur 2:: ein elektrochirurgisches Instrument;
- Figur 3:: ein schematisches Schaltbild eines Elektrochirurgie-Generators;
- Figur 4:: ein schematisches Bild eines Prozessors in Verbindung mit einem Generator-Datenspeicher des Elektrochirurgie-Generators aus Figur 3
- Figur 5:: ein schematisches Bild einer alternativen Konfiguration des Prozessors in Verbindung mit einem Generator-Datenspeicher des Elektrochirurgie-Generators aus Figur 3; und
- Figur 6:: ein Beispiel für eine Definition von in dem elektrochirurgischen Instrument zu speichernden Nutzungs- und Zustandsdaten.

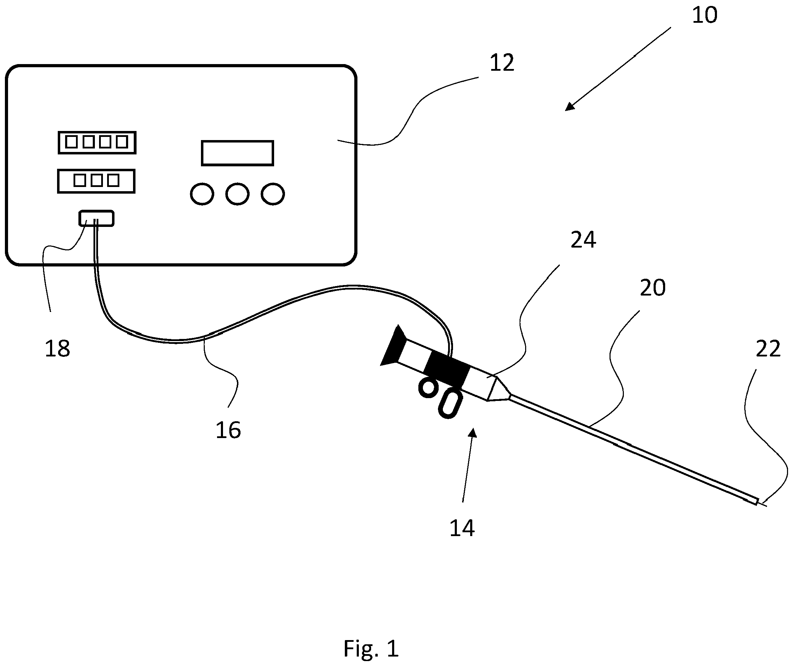

In Figur 1 ist ein Elektrochirurgiesystem 10 abgebildet. Das Elektrochirurgiesystem 10 umfasst einen Elektrochirurgie-Generator 12 und ein elektrochirurgisches Instrument 14. Das elektrochirurgisches Instrument 14 ist über ein Anschlusskabel 16 mit elektrischen Aus- und Eingängen 18 des Elektrochirurgie-Generators 12 verbunden.

Das elektrochirurgische Instrument 14 weist einen Schaft 20 auf, an dessen Ende sich eine aktive Elektrode 22 befindet. Der Schaft 20 ist an einem Griff 24 des elektrochirurgischen Instruments 14 befestigt.

Das elektrochirurgische Instrument 14 weist einen beschreibbaren, vorzugsweise nichtflüchtigen Instrumenten-Datenspeicher 26 auf. Der Instrumenten-Datenspeicher 26 kann beispielsweise ein EEPROM (electrically erasable programmable read-only memory) sein. Ein EEPROM ist ein nichtflüchtiger Datenspeicher, der gelesen, beschrieben und schreibgeschützt werden kann. Der Datenspeicher 26 ist beispielsweise in dem Handgriff 24 des elektrochirurgischen Instruments 14 untergebracht.

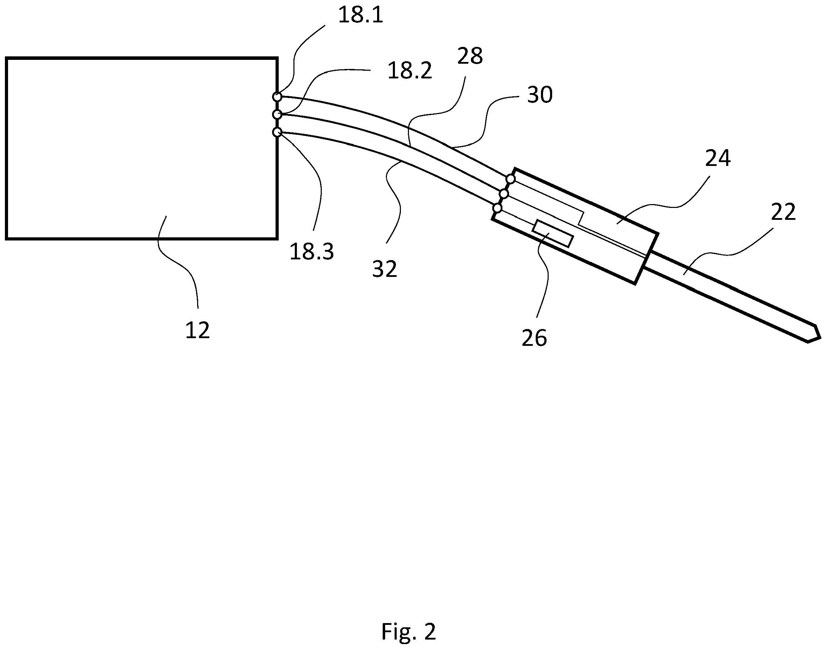

In dem Anschlusskabel 16 befinden sich sowohl Versorgungsleitungen 28 und 30 als auch wenigstens eine Datenleitung 32 zum Auslesen von Daten aus dem Datenspeicher 26 sowie zum Schreiben von Daten in den Instrumenten-Datenspeicher 26. Die Versorgungsleitungen 28 verbinden die aktive Elektrode 22 und eine nicht weiter dargestellte neutrale Elektrode mit den elektrischen Ausgängen 18.1 und 18.2 des Elektrochirurgie-Generators 12. Über die Datenleitung 32 ist der Instrumenten-Datenspeicher 26 mit einem entsprechenden Anschluss 18.3 des Elektrochirurgie-Generators 12 verbunden. Dies ist schematisch in Figur 2 dargestellt.

Die Datenleitung 32 in dem Anschlusskabel 16 sowie auch der Anschluss 18.3 können mehradrig bzw. mehrpolig sein. Zusätzlich zu der Datenleitung 32 oder anstelle der Datenleitung 32 kann auch eine drahtlose Schnittstelle für die Datenübertragung von dem elektrochirurgischen Instrument 14 zu dem Elektrochirurgie-Generator 12 vorgesehen sein. Eine solche drahtlose Schnittstelle kann beispielsweise eine Bluetooth Schnittstelle, eine NFC Schnittstelle oder eine RFID Schnittstelle sein.

Der Datenspeicher 26 des elektrochirurgischen Instruments 14 ist partitioniert und weist insbesondere eine einen Schreib-Lese-Bereich (Read-Write-Area) bildende Partition auf, von der die Nutzungs- und Zustandsdaten gelesen oder auf die Nutzungs- und Zustandsdaten geschrieben werden können. Das Lesen und Schreiben der Nutzungs- und Zustandsdaten erfolgt über eine Datenallokationstabelle (Data Allocation Table). Die Datenallokationstabelle ist vorzugsweise in einem Generator-Datenspeicher 72 des Elektrochirurgie-Generators 12 zum Betreiben des elektrochirurgischen Instruments 14 gespeichert.

In der den Schreib-Lese-Bereich (Read-Write-Area) bildenden Partition des Datenspeichers 26 zu speichernde Nutzungs- und Zustandsdaten können z. B. Aktivierungs- und Steckzeiten, Aktivierungsenergien, Fehlerzustände oder verbleibende Aktivierungszyklen sein. Diese Nutzungs- und Zustandsdaten sollen vollständig auf dem elektrochirurgischen Instrument 14 hinterlegt werden, so dass auch nach Markteinführung eines Elektrochirurgie-Generators, Anpassungen der zu speichernden Nutzungs- und Zustandsdaten über die Programmierung des elektrochirurgischen Instruments 14 möglich sind. Der Instrumenten-Datenspeicher 26 kann partitioniert sein. In diesem Fall muss sich das Speichermodell in die gegebene Partitionierung des Datenspeichers 26 des elektrochirurgischen Instruments 14 einfügen. Diese ist in der nachfolgenden Tabelle 1 dargestellt. Konkret steht ein Bereich definierbarer Größe zur Verfügung, die so genannte Read-Write-Area, in der die Nutzungs- und Zustandsdaten abgelegt werden können.

**Tabelle 1**

| | **Bereich** | **Beschreibung** |
|---|---|---|
| 1 | Legacy Tables | Modeinformationen für Altsysteme |
| 2 | Config Area | Beschreibung der nachfolgenden Bereiche (z. B. Größenangaben) |
| 3 | Read-only Area | Strukturierte Daten, die neben dem Betriebsmodus vor allem auch die zu speichernden Zustands- und Nutzungsdaten definieren |
| 4 | Write-Protect Block Alignment | Chip-spezifische Anhangdaten für den Lesedatenbereich |
| **5** | **Read-write Area** | **Zustands- und Nutzungsdaten zum Austausch zwischen Instrument und Elektrochirurgiegenerator** |
| 6 | Write-Protect Block Alignment | Chip-spezifische Anhangdaten für den Lese- und Schreibdaten-Bereich |
| 7 | Next table(s) | Speicherplatz für weitere Modetabellen |
| 8 | Last Config Area | Endekonfiguration |

Der Schreib-Lese-Bereich (Read-Write-Area) ist ein Bereich für das erfindungsgemäße Speichermodell, um Zustands- und Nutzungsdaten speichern zu können.

Um die oben genannten Anforderungen zu erfüllen, ist für den Instrumenten-Datenspeicher 26 des elektrochirurgischen Instruments 14 ein Speichermodell vorgesehen, das durch eine Datenallokationstabelle (Data Allocation Table) definiert ist, wie sie in der nachfolgenden Tabelle 2 dargestellt ist. Die Datenallokationstabelle muss allen Lese- und Schreibergeräten bekannt sein, ist gemäß dem Ausführungsbeispiel aber nicht auf dem Instrumenten-Datenspeicher 26 des elektrochirurgischen Instruments 14 hinterlegt. Vorzugsweise ist die Datenallokationstabelle in dem Generator-Datenspeicher 72 des Elektrochirurgie-Generators 12 gespeichert.

**Tabelle 2: Exemplary Data Allocation Table**

| **Name** | **ID** | **Beschreibung** | **Größe [Byte]** | **Typ** |
|---|---|---|---|---|
| NONE | 0 | Keine Daten | 0 | N/A |
| RTOU | 1 | Zähler #1 für Ultraschallaktivierungen | 2 | 2 byte unsigned integer counter |
| TIMESTAMP | 2 | Zeitstempel (z.B. letzte Steckzeit) | 4 | 4 byte unsigned integer timestamp |
| ENERGY | 3 | Energie (z. B. abgegebene Energie der letzten Aktivierung) | 4 | 4 byte unsigned integer energy |
| ACTIVATIONTIME | 4 | Aktivierungszeit (z. B. Zeit der letzten Aktivierung) | 4 | 4 byte unsigned integer number |
| GENERIC | 5 | Allgemeine Daten | 4 | 4 byte float value |
| ACTIVATION_COUNT | 6 | Zähler #2 für Ultra-schallaktivierungen | 4 | 4 byte unsigned integer counter |
| RTOU steht für "remaining times of use" | | | | |

Konkret definiert die Data Allocation Table verschiedene Bezeichner (Tags), die wiederum
- die Größe der nachfolgenden Daten (z. B. 4 Byte) und
- den Typ der nachfolgenden Daten (z. B. Zeitstempel)

### definieren.

Die auszutauschenden und zu speichernden Daten können mit einer Programmierschnittstelle 82 (siehe weiter hinten im Text) definiert und schließlich in dem Schreib-Lese-Bereich (Read-Write-Area) des Instrumenten-Datenspeichers 26 des elektrochirurgischen Instruments 14 als Folge von *Bezeichner (Tag)* und *Wert* gespeichert werden. Die Summe der Daten ergibt die Größe des Schreib-Lese-bereichs (Read-Write-Area) und wird vorzugsweise bei der Programmierung und Partitionierung des Instrumenten-Datenspeichers 26 des elektrochirurgischen Instruments 14 in dessen Konfigurationsbereich (Config-Area) abgespeichert. Der Elektrochirurgie-Generator 12 kann die Daten des Schreib-Lese-Bereiches auslesen. Die Bezeichner (Tags) definieren, welche Daten der Elektrochirurgie-Generator 12 auslesen und verarbeiten soll und welche Daten er aktualisieren soll.

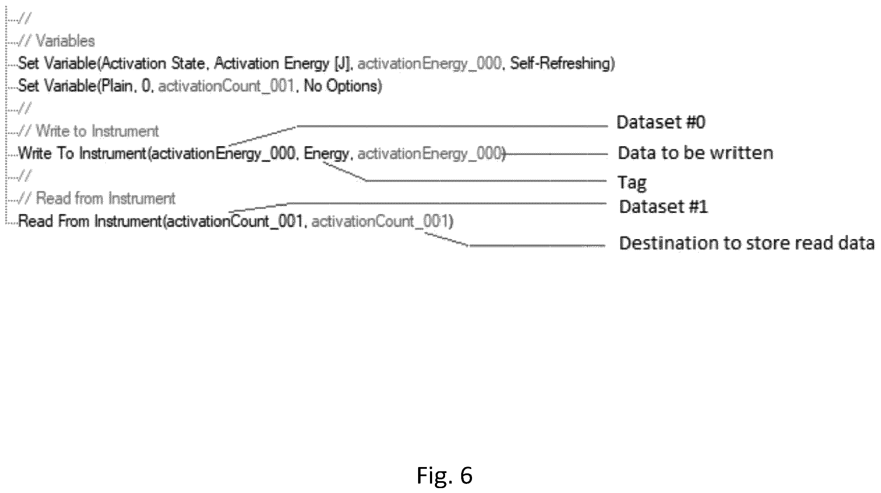

Figur 6 zeigt ein Beispiel für eine Definition von Nutzungs- und Zustandsdaten mittels einer weiter unten beschriebenen Programmierschnittstelle 82

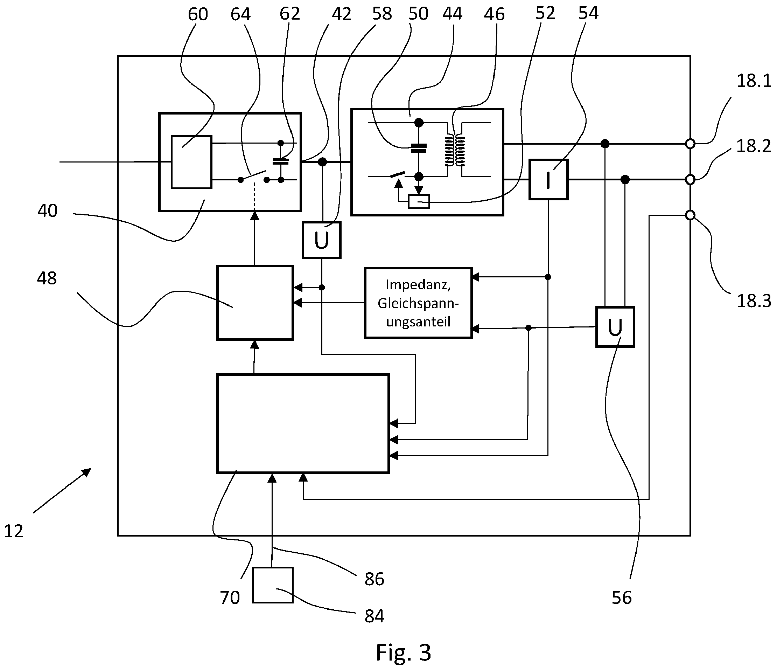

Die zum Betreiben des elektrochirurgischen Instruments 14 an dessen aktive Elektrode 22 und eine Gegenelektrode abzugebende Ausgangswechselspannung wird im Betrieb von dem Elektrochirurgie-Generator 12 bereitgestellt. Wie Figur 3 zu entnehmen ist, weist der Elektrochirurgie-Generator 12 hierzu ein Hochspannungsnetzteil 40 auf, das beispielsweise an das übliche öffentliche Stromversorgungsnetz angeschlossen werden kann und an seinem Ausgang 42 einen Hochspannungsgleichstrom mit einer Ausgangsgleichspannung bereitstellt. Dieser Ausgangsgleichstrom wird einem Hochfrequenzteil 44 des Elektrochirurgie-Generators 12 zugeführt. Der Hochfrequenzteil 44 des Elektrochirurgie-Generators 12 wirkt als Wechselrichter und erzeugt eine hochfrequente Ausgangswechselspannung, die über einen Ausgangstransformator 46 des Hochfrequenzteils 44 an die Ausgänge 18.1 und 18.2 des Elektrochirurgie-Generators 12 abgegeben wird. An die Ausgänge 18.1 und 18.2 des Elektrochirurgie-Generators 12 kann das elektrochirurgische Instrument 14 angeschlossen werden, wie es in den Figuren 1 und 2 dargestellt ist.

Um die Ausgangswechselspannung des Elektrochirurgie-Generators 12 zu steuern, ist eine Generatorsteuereinheit 48 vorgesehen, die die Ausgangswechselspannung an den Ausgängen 18.1 und 18.2 des Elektrochirurgie-Generators 12 auf Basis eines Ausgangswechselspannungsmaximalwertes derart steuert, dass beispielsweise ein eingestellter Ausgangsspannungsmaximalwert im Betrieb nicht überschritten wird.

Die Ausgangswechselspannung des Elektrochirurgie-Generators 12 - und damit auch der Ausgangswechselstrom und die Ausgangsleistung - können von einer Generatorsteuereinheit 48 gesteuert werden.

Die Generatorsteuereinheit 48 steuert das Hochspannungsnetzteil 40 in Abhängigkeit von durch einen jeweiligen Betriebsmodus definierten Maximalwerten und von im Betrieb von Erfassungseinheiten 54, 56 und 58 erfassten aktuellen Werten der Ausgangswechselspannung, der Ausgangsspitzenspannung, des Ausgangswechselstroms, des Gleichspannungsanteil an der Ausgangswechselspannung oder der Ausgangsgleichspannung oder einer Kombination von Werten dieser Parameter.

Die konkreten Maximalwerte und der zeitliche Ablauf für das Erzeugen der Ausgangswechselspannung des Elektrochirurgie-Generators 12 und deren Abhängigkeit von erfassten Momentanwerten hängen vom jeweiligen Betriebsmodus (Mode) ab, in dem der Elektrochirurgie-Generator 12 gerade betrieben wird.

Ein Betriebsmodus wird beispielsweise durch Betätigen eines entsprechenden Schalters, beispielsweise einem Fußschalter 84 durch einen Nutzer aufgerufen.

In einem jeweiligen Betriebsmodus wird der Betrieb des Elektrochirurgie-Generators 12 von einem Prozessor 70 in Verbindung mit einem Betriebsprogram 74 gesteuert, das in dem Generator-Datenspeicher 72 gespeichert ist. Der Prozessor 70 bildet - gesteuert durch das Betriebsprogramm 74 - beispielsweise die Maximalwerte für die verschiedenen Betriebsparameter, wie die Ausgangswechselspannung, den Ausgangswechselstrom, die Ausgangsleistung oder aber auch den Gleichspannungsanteil an der Ausgangswechselspannung, deren jeweils aktuelle Werte während des Betriebs des Elektrochirurgie-Generators 12 erfasst werden.

Der Prozessor 70 ist mit dem Generator-Datenspeicher 72 verbunden, in dem das Betriebsprogramm 74 für den Elektrochirurgie-Generator 12 hinterlegt ist.

Während des Ablaufs des in dem Generator-Datenspeicher 72 hinterlegten Betriebsprogramms 74 greift der Prozessor 70 an in dem Betriebsprogramm 74 hinterlegten Stellen auf für einen jeweiligen Betriebsmodus in dem Generator-Datenspeicher 72 hinterlegte Betriebsvorgaben 76 wie beispielsweise Daten, die Werte für Betriebsparameter repräsentieren und/oder Steuerbefehle zurück. Die Betriebsvorgaben 76 geben beispielsweise bestimmte Werte für die Ausgangsgleichspannung des Hochspannungsnetzteils oder die Ausgangswechselspannung, den Ausgangswechselstrom des Hochfrequenzteils oder Ähnliches vor. In dem Generator-Datenspeicher 72 hinterlegte Betriebsvorgaben 76 schließen aber auch bestimmte Steuerbefehle ein, wie beispielsweise "*if"* oder *"while"* oder *"true"* oder *"false".* Auf diese Weise können die als Betriebsvorgaben 76 hinterlegten Daten und Steuerbefehle mittels Verweisen in einer Datenstruktur 78 beispielsweise zu solchen Steueranweisungen kombiniert werden wie *"vergleiche den aktuellen Wert der Ausgangswechselspannung mit dem Betrag 200 und gib ein "true" aus, wenn der aktuelle Wert der Spannung kleiner oder gleich 200 ist und ein "false", wenn der aktuelle Wert größer ist als 200".* Andere Daten und Steuerbefehle können beispielsweise die Steueranweisung ergeben, dass die maximale Ausgangsgleichspannung des Hochspannungsnetzteils 100 Volt sein soll.

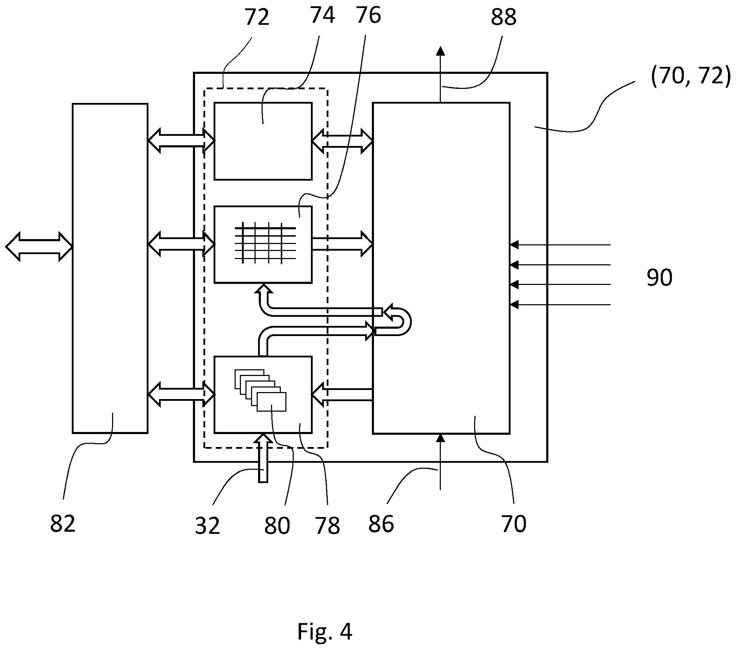

Im Betrieb greift der Prozessor 70 nicht direkt auf die Betriebsvorgaben 76 zu, sondern ruft an entsprechenden Stellen des Betriebsprogramms die Datenstruktur 78 auf, in der für einen jeweiligen Betriebsmodus Verweise hinterlegt sind, die auf entsprechende Betriebsvorgaben 76 verweisen, siehe z.B. Figur 4. Die Verweise können beispielsweise Zahlen sein, insbesondere 1-Byte große Binärzahlen, da diese wenig Speicherplatz erfordern. In diesem Falle können die Betriebsvorgaben 76 nach Art einer Tabelle aufgebaut sein, in der jedem Verweis (also beispielsweise jeder Hexadezimalzahl) die entsprechenden Steuerbefehle oder Daten zugeordnet sind.

Um verschiedene Betriebsmodi zu verwirklichen, sind in der Datenstruktur 78 eine Vielzahl von Datensätzen 80 hinterlegt, von denen jeder ein oder mehrere Verweise enthält, denen aufgrund der Struktur des jeweiligen Datensatzes - insbesondere der Reihenfolge, in der die Verweise gespeichert sind - Zeilennummern zugeordnet werden können. Die Zeilennummern können Adressen innerhalb der strukturierten Daten eines Datensatzes sein, auf die der Prozessor 70 durch das Betriebsprogramm gesteuert zugreifen kann. Die einer Zeilennummer zugeordneten Verweise verweisen auf konkrete Betriebsvorgaben 76 in dem Generator-Datenspeicher 72 und veranlassen den Prozessor 70, die entsprechenden Betriebsvorgaben aus dem Generator-Datenspeicher 72 auszulesen, nachdem der Prozessor 70 zuvor auf die in der Datenstruktur 78 hinterlegte und die zugehörige Zeilennummer bezeichnete Einsprungadresse zugegriffen hat. Die Betriebsvorgaben 76 können Steueranweisungen, Steuerbefehle oder Parameterwerte sein, welche das Betriebsprogramm 74 an der jeweiligen Stelle des Betriebsprogramms anwenden soll, an der sich in dem Betriebsprogramm 74 ein Verweis auf eine Zeilennummer in der Datenstruktur 78, befindet.

In der Datenstruktur 78 sind Verweise auf Betriebsvorgaben 76 in dem Generator-Datenspeichers 72 in einer geordneten Sequenz abgespeichert. Die Struktur eines jeweiligen Datensatzes in der Datenstruktur 78 erlaubt es, den Verweisen Zeilennummern nach Art von Adressen innerhalb der Datenstruktur 78 zuzuweisen, damit beispielsweise auch Sprünge oder Rücksprünge auf Verweise in der Datenstruktur 78 möglich sind und nicht lediglich ein streng sequentielles Abarbeiten der Verweise durch das Betriebsprogramm. Zeilennummern können als Einsprungadressen innerhalb der strukturierten Daten in der Datenstruktur 78 dienen, auf die der Prozessor 70 durch das Betriebsprogramm gesteuert zugreift. Die den Zeilennummern zugeordneten Verweise verweisen auf entsprechende Betriebsvorgaben in dem Generator-Datenspeicher 72. Die Betriebsvorgaben können Steuervorgaben, nämlich beispielsweise einzelne Steuerbefehle, zusammengesetzte Steueranweisungen oder Parameterwerte sein. Die Steuervorgaben steuern den Betrieb des Elektrochirurgie-Generators 12 in dem jeweiligen Betriebsmodus. Die Steuervorgaben können auch Steuerbefehle sein, die den Aufruf anderer Verweise in der Datenstruktur 78 bewirken. Allerdings ist dies nur innerhalb eines Datensatzes mit strukturierten Daten möglich, die zu einem jeweiligen Betriebsmodus gehören.

Die parametrisierten Verweise sind vorzugsweise durch Hexadezimalzahlen repräsentiert, die zusammen strukturierte Daten eines Datensatzes 80 bilden. Ein Datensatz gehört zu einem Betriebsmodus und kann zum Beispiel folgendes enthalten:

| | | |
|---|---|---|
| *006F* | **11** | |
| *0070* | **36** | 02 30 00 C8 00 |
| *0076* | **12** | |
| *0077* | **0F** | 04 64 00 |
| *0078* | **56** | |
| *007C* | **4B** | 03 02 |
| *007F* | **13** | |

Die kursiv dargestellten Zahlen sind beim Auslesen des Datensatzes 80 generierte Zeilennummern und sind nicht in dem Datenspeicher 26 des elektrochirurgischen Instrument 14 gespeichert, sondern werden von dem Betriebsprogramm entsprechend der Reihenfolge der Verweise in einem jeweiligen Datensatz selbst erzeugt. Die Zeilennummern ergeben sich aus der Reihenfolge der Verweise (also deren Struktur) in einem jeweiligen Datensatz. Die im Beispiel als kursiv dargestellte Zahlen dargestellten Zeilennummern sind einfach aufsteigende Zahlen entsprechend der Länge der strukturierten Daten. Die fett dargestellten Zahlen dienen als Verweise (oder Zeiger bzw. Pointer), die jeweils auf eine konkrete Betriebsvorgabe 76 in dem Generator-Datenspeicher 72 verweisen, und zwar in dem dargestellten Beispiel auf Steuerbefehle. So verweist z.B. "11" auf den Steuerbefehl "IF", "36" auf einen Vergleich, der durch die nachfolgenden, zugeordneten Zahlen "02 30 00 C8 00" spezifiziert wird, "12" auf den Steuerbefehl "THEN", "0F" auf einen Steuerbefehl zum Setzen eines durch die nachfolgenden, zugeordneten Zahlen "04 64 00" spezifizierten Parameterwertes und "56" auf den Steuerbefehl "ELSE".

Aus der jeweiligen mittels parametrisiertem Verweis aufgerufenen Betriebsvorgabe 76 ergibt sich auch, welche Zusatzinformationen (wie z.B. "02 30 00 C8 00" in dem obigen Beispiel) noch relevant sind.

Das Betriebsprogramm 74 kann das vorstehend dargestellte Beispiel wie folgt lesen und übersetzen:
**11** - if -> keine Zusatzinfos erforderlich
**36** - Vergleich -> 5 Zeichen an Zusatzinformationen erforderlich (02 = 1 Zeichen Vergleichsart, 30 00 = 2 Zeichen Zahl 1, C8 00 = 2 Zeichen Zahl 2) **12** - then -> keine Zusatzinfos erforderlich
**0F** - Ausgangswert setzen -> 3 Zeichen Zusatzinformationen (04 = 1 Zeichen Welcher Ausgangswert; 64 00 = 2 Zeichen Setzwert)

Übersetzt kann dies bedeuten "*Vergleiche, ob Zahl 1 (30 00) größer ist, als Zahl 2 (C8 00). Wenn das Ergebnis TRUE ist, dann setze Spannung auf 100 V (64 00), ansonsten* ..."

Die in dem Beispiel als Hexadezimalzahlen dargestellten Binärzahlen (und hier verkürzt als "Hexadezimalzahlen" bezeichneten Zahlen) in einem jeweiligen Datensatz 80 repräsentieren somit zum einen Verweise, auf deren Basis das Betriebsprogramm 74 auf konkrete Betriebsvorgaben 76 in dem Generator-Datenspeicher 72 zugreifen kann. Die in einem Datensatz strukturiert (geordnet) abgelegten Hexadezimalzahlen repräsentieren die vorgenannten Verweise auf in dem Generator-Datenspeicher 72 gespeicherte Betriebsvorgaben 76 wie Steuerbefehle und Parameter, denen aufgrund der Struktur des Datensatzes 80 Zeilennummern zugeordnet werden können, die selbst nicht explizit im Datensatz 80 gespeichert sein müssen, sondern von dem Betriebsprogramm 74 beim Einlesen eines jeweiligen Datensatzes 80 erzeugt werden können.

Die in einem jeweiligen Datensatz 80 gespeicherten und in dem Beispiel als Hexadezimalzahlen dargestellten Binärzahlen dienen als Pointer (oder Zeiger), die jeweils auf eine konkrete Betriebsvorgabe 76 in dem Generator-Datenspeicher 72 des Elektrochirurgie-Generators 12 verweisen, so dass die entsprechende Hexadezimalzahl (d.h. der Verweis in den strukturierten Daten) mit einer entsprechenden Betriebsvorgabe 76 in dem Generator-Datenspeicher 72 des Elektrochirurgie-Generators 12 verknüpft ist. Diese Hexadezimalzahlen werden somit zur Bezeichnung einer entsprechenden Betriebsvorgabe 76 in dem Generator-Datenspeicher 72 des Elektrochirurgie-Generators 12 verwendet. Diese Hexadezimalzahlen sind somit eine Art Pointer, um das Betriebsprogramm zu Betriebsvorgaben 76 in dem Generator-Datenspeicher 72 des Elektrochirurgie-Generators 12 zu leiten, wo das Betriebsprogramm 74 jeweils eine Betriebsvorgabe für das Betriebsprogramm 74 während seiner Ausführung abrufen kann. Der Aufruf der Verweise erfolgt durch das Betriebsprogramm 74 gesteuert und führt dazu, dass das Betriebsprogramm 74 diejenigen Betriebsvorgaben 76 in dem Generator-Datenspeicher 72 abruft, auf die die Verweise verweisen. Dabei sind auch Sprünge innerhalb der Verweise möglich, die durch ihre Sequenz oder Zeilennummern identifiziert sind. Die strukturierten Daten in einem Datensatz 80 können so über die ihnen entsprechenden Speichereinträge in dem Generator-Datenspeicher 72 in konkrete Betriebsvorgaben 76 für das Betriebsprogramm 74 übersetzt werden.

In dem vorstehend beispielhaft wiedergegebenen Datensatz stellen die Einträge in der ersten Spalte *(*"*006F, 0070, 0076*...") die Zeilennummern dar, auf die das Betriebsprogramm 74 verweisen kann. Die Zeilennummern sind nicht in dem Datenspeicher 26 des elektrochirurgischen Instrument 14 gespeichert, sondern werden von dem Betriebsprogramm 74 selbst erzeugt, da die Zeilennummern einfach aufsteigende Zahlen entsprechend der Länge der strukturierten Daten sind:
*0070* 36 02 30 00 C8 00 (Länge der Daten = 6, d.h. nächste Adresse ist *0076)*
*0076* 12 (Länge der Daten = 1, d. h. nächste Adresse ist *0077)*

Die Einträge in jeder Zeile ("11, 36 02 30 00 C8 00, 12...") verweisen auf Positionen von Speichereinträgen in dem Generator-Datenspeicher 72, die Betriebsvorgaben 76 enthalten. Der durch 11 bezeichnete Eintrag in dem Generator-Datenspeicher 72 kann zum Beispiel eine Betriebsvorgabe sein, die eine "IF"-Anweisung repräsentiert, während der durch 12 bezeichnete Eintrag eine "THEN"-Anweisung sein kann. "IF"-Anweisung und "THEN"-Anweisung sind jeweils eine Betriebsvorgabe. Die in den strukturierten Daten des abgebildeten Datensatzes zwischen 11 und 12 stehenden Hexadezimalzahlen "36 02 30 00 C8 00" können aus den dazugehörigen Betriebsvorgaben 76 in dem Generator-Datenspeicher 72 in eine Steueranweisung übersetzt werden, wie zum Beispiel *"Vergleiche den letzten abgelesenen Wert der Spannung (30 00) mit der Zahl 200 (C8 00), und wenn die Spannung kleiner oder gleich 200 ist, ist das Ergebnis "TRUE", andernfalls ist das Ergebnis "FALSE"'.* Wenn das Betriebsprogramm 74 an der (vom Betriebsprogramm 74 erzeugten) Adresse "*0077*" die Speichereinträge für die Zeichenfolge "**0F** 04 64 00" aus dem Generator-Datenspeicher 72 abruft, könnte dies eine Einstellung für den Elektrochirurgie-Generator 12 bezeichnen; die Einstellung kann z.B. sein, dass ein Maximalwert für die Ausgangsgleichspannung (0F 04) auf 100V (64 00) eingestellt ist.

Der Betrieb des Elektrochirurgie-Generators 12 in einem jeweiligen Betriebsmodus hängt somit zum einen von dem in dem Generator-Datenspeicher 72 gespeicherten Betriebsprogramm 74 ab. Darüber hinaus hängt das Betriebsverhalten des Elektrochirurgie-Generators 12 in einem jeweiligen Betriebsmodus aber auch von dem für einen jeweiligen Betriebsmodus aufgerufenen Datensatz 80 in der Datenstruktur 78 ab sowie von den ebenfalls in dem Generator-Datenspeicher 72 gespeicherten Betriebsvorgaben 76.

Der Vorteil eines solchen Elektrochirurgie-Generators 12 ist, dass neue Betriebsmodi leicht durch Erzeugen neuer Datensätze 80 in der Datenstruktur 78 definiert werden können und beispielsweise ein einzelner Betriebsmodus allein durch Ändern des entsprechenden Datensatzes 80 in der Datenstruktur 78 geändert werden kann, ohne dass das Betriebsprogramm 74 in dem Generator-Datenspeicher 72 oder die Betriebsvorgaben 76 in dem Generator-Datenspeicher 72 geändert werden müssen. Auf der anderen Seite können globale Parameter, wie beispielsweise die möglichen zur Verfügung stehenden Steueranweisungen oder vom Elektrochirurgie-Generator 12 abhängigen Betriebsparameter, wie dessen maximale Ausgangswechselspannung oder eine minimal zulässige Ausgangsgleichspannung als Betriebsvorgaben 76 in dem Generator-Datenspeicher 72 hinterlegt sein und dort auch gegebenenfalls zentral für alle möglichen Betriebsmodi zugleich geändert werden.

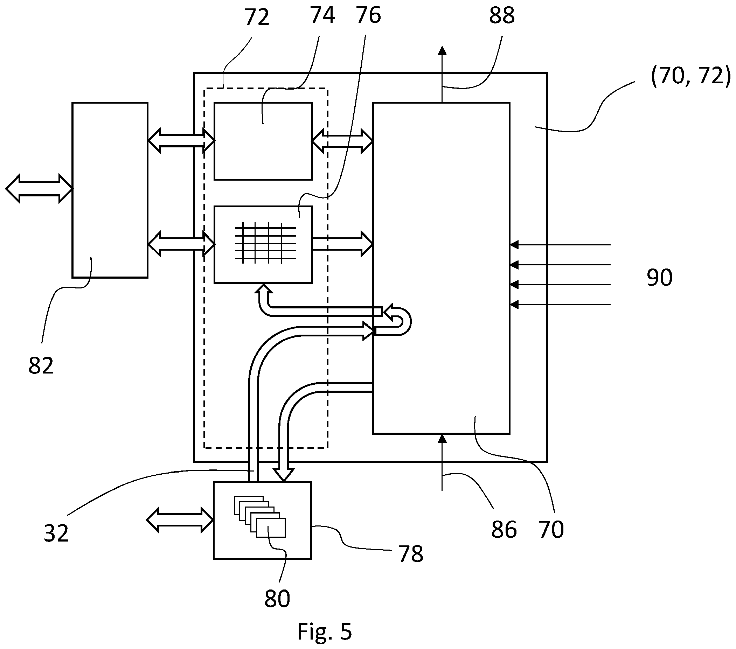

Ein weiterer Vorteil des Elektrochirurgie-Generators 12 ist, dass ein Datensatz 80, dessen strukturierte Daten indirekt einen für das elektrochirurgische Instrument 14 geeigneten Betriebsmodus definieren, auch in einem elektrochirurgischen Instrument 14 hinterlegt sein kann; siehe Figur 5. Konkret kann der Instrumenten-Datenspeicher 26 des elektrochirurgischen Instruments 14 einen Datensatz 80 mit strukturierten Daten enthalten, die zu der Datenstruktur 78 kompatibel sind und genau wie andere strukturierte Daten in der Datenstruktur 78 durch entsprechende Verweise auf konkrete Betriebsvorgaben 76 in dem Generator-Datenspeicher 72 indirekt einen jeweiligen Betriebsmodus definieren. In dem Instrumenten-Datenspeicher 26 sind dann beispielsweise in einem Nur-Lese-Bereich ein oder mehrere Datensätze 80 mit strukturierten Daten gespeichert und ein einem Schreib-Lese-Bereich sind Zustands- und Nutzungsdaten für ein jeweils individuelles elektrochirurgisches Instrument 14 gespeichert.

Der Elektrochirurgie-Generator 12 ist so konfiguriert, dass er beim Anschließen eines elektrochirurgischen Instruments 14 jeweils - soweit vorhanden - zunächst dessen Instrumenten-Datenspeicher 26 ausliest und die strukturierten Daten aus dem in dem Datenspeicher 26 gespeicherten Datensatz in die Datenstruktur 78 einträgt. Auf diese Weise steht dem Elektrochirurgie-Generator 12 dann im Betrieb ein genau auf das jeweilige elektrochirurgische Instrument 14 zugeschnittener Betriebsmodus zur Verfügung. Gleichzeitig liest der Elektrochirurgie-Generator 12 auch die Zustands- und Nutzungsdaten aus dem Schreib-Lese-Bereich des Instrumenten-Datenspeichers 26 des elektrochirurgischen Instruments 14 aus.

Um einen Zugriff auf die Inhalte des Instrumenten-Datenspeichers 26 des elektrochirurgischen Instruments 14 zu erlauben, ist wenigstens die Datenleitung 22 mit einem zugehörigen Anschluss 18.3 vorgesehen. Alternativ oder zusätzlich kann auch eine drahtlose Schnittstelle wie beispielsweise eine Bluetooth Schnittstelle oder eine NFC Schnittstelle für den Zugriff auf die Inhalte des Instrumenten-Datenspeichers 26 des elektrochirurgischen Instruments 14 vorgesehen sein.

Beim Übertragen der strukturierten Daten von dem Instrumenten-Datenspeicher 26 des elektrochirurgischen Instruments 14 in einem entsprechenden Datensatz in der Datenstruktur 78 des Elektrochirurgie-Generators 12 können ggf. die durch die Struktur der strukturierten Daten in dem Instrumenten-Datenspeicher 26 vorgegebenen Zeilennummern erzeugt werden, die zu dem Betriebsprogramm des Elektrochirurgie-Generators 12 passen.

Von großem Vorteil ist, dass der in dem Nur-Lese-Bereich des Instrumenten-Datenspeichers 26 gespeicherte Datensatz 80 nur strukturiert geordnete Verweise (Pointer auf weitere Speichereinträge 76 in dem Generator-Datenspeicher 72) enthält, jedoch nicht unmittelbar irgendwelche Steueranweisungen oder Betriebsparameter für einen jeweiligen Betriebsmodus, da die Steueranweisungen und die Betriebsparameter als Betriebsvorgaben 76 in dem Generator-Datenspeicher 72 des Elektrochirurgie-Generators 12 zentral gespeichert sind.

Alternativ kann der Elektrochirurgie-Generator 12 auch so konfiguriert sein, dass er während des Betriebs - also während des Ablaufs des Betriebsprogramms 74 - den Instrumenten-Datenspeicher 26 des elektrochirurgischen Instruments direkt 14 ausliest. Dies ist bei dem in Figur 5 dargestellten Beispiels der Fall. In diesem Fall ist es nicht nötig, dass die strukturierten Daten des Datensatzes in dem Datenspeicher 26 des elektrochirurgischen Instruments 14 zunächst in die Datenstruktur 78 auf dem Elektrochirurgie-Generator 12 übertragen werden. Die Zeilennummern müssen dann allerdings zu den entsprechenden Aufrufen in dem Betriebsprogramm und den Betriebsvorgaben repräsentierenden Einträgen in dem Generator-Datenspeicher 72 passen. Zustands- und Nutzungsdaten aus dem Schreib-Lese-Bereich des Instrumenten-Datenspeichers 26 werden allerdings vorzugsweise immer kurz nach dem Anschließen eines elektrochirurgischen Instruments an den Elektrochirurgie-Generator 12 von diesem ausgelesen.

Ein Vorteil eines Elektrochirurgie-Systems 10 der hier beschriebenen Art ist, dass verschiedene, den Betrieb des Elektrochirurgie-Generators 12 bestimmende Daten unabhängig voneinander gepflegt werden können. So ist das in dem Generator-Datenspeicher 72 gespeicherte Betriebsprogramm 74 unabhängig von den Betriebsvorgaben 76 in dem Generator-Datenspeicher 72 gespeichert. Letztere sind wiederum unabhängig von den strukturierten Daten in der Datenstruktur 78 gespeichert.

Zu programmieren des Betriebsprogramms 74, der Betriebsvorgaben 76 und der Datenstruktur 78 mit den strukturierten Daten eine Programmierschnittstelle 82 vorgesehen, die vorzugsweise so konfiguriert ist, dass sie unterschiedlichen Nutzern unterschiedliche Rechte einräumt. So können die Rechte für das Programmieren des Betriebsprogramms 74, das in dem Generator-Datenspeicher 72 gespeichert ist, für das Eingeben der Betriebsvorgaben 76, die ebenfalls in dem Generator-Datenspeicher 72 gespeichert sind und für die strukturierten Daten, die in der Datenstruktur 78 in dem Generator-Datenspeicher 72 und/oder dem Instrumenten-Datenspeicher 26 gespeichert sind, unterschiedlich vergeben werden. Insbesondre kann auf diese Weise sichergestellt werden, dass Änderungen an den Betriebsvorgaben 76 oderÄnderungen an dem Betriebsprogramm 74 nur von Entwicklern vorgenommen werden können, die mit dem jeweiligen Elektrochirurgie-Generator 12 vertraut sind. Die Programmierung des Betriebsprogramms 74 und der Betriebsvorgaben 76 kann somit durch Entwickler erfolgen, die mit dem jeweiligen Elektrochirurgie-Generator 12 vertraut sind, während die Definition von Betriebsmodi für ein elektrochirurgisches Instrument 14 durch Schaffen eines entsprechenden Datensatzes 80 mit strukturierten Daten durch einen mit dem elektrochirurgischen Instrument 14 vertrauten Entwickler erfolgen kann. Vorzugsweise werden von einem mit dem elektrochirurgischen Instrument 14 vertrauten Entwickler erzeugte Datensätze in dem Instrumenten-Datenspeicher 26 des entsprechenden elektrochirurgischen Instruments 14 gespeichert, während weitere Betriebsmodi auch direkt in der Datenstruktur 78 auf dem Elektrochirurgie-Generator 12 gespeichert werden können. Hierzu kann der Elektrochirurgie-Generator 12 beispielsweise eine USB-Programmierschnittstelle aufweisen.

Für in dem Instrumenten-Datenspeicher 26 des entsprechenden elektrochirurgischen Instruments 14 gespeicherte strukturierte Daten und Zustands- und Nutzungsdaten steht entweder die Datenleitung 32 in dem Anschlusskabel 16 mit entsprechender Schnittstelle zur Verfügung - oder, alternativ oder zusätzlich - auch eine drahtlose Schnittstelle wie beispielsweise eine Bluetooth Schnittstelle oder eine NFC Schnittstelle. Die strukturierten Daten aus dem Datensatz 80 sowie die Zustands- und Nutzungsdaten in dem Instrumenten-Datenspeicher 26 des elektrochirurgischen Instruments 14 können dann beim Anschließen des elektrochirurgischen Instruments 14 in die Datenstruktur 78 auf dem Elektrochirurgie-Generator übertragen werden.

Dies ist insbesondere in Bezug auf verschiedene elektrochirurgische Instrumente 14 von Bedeutung, da die elektrochirurgischen Instrumente 14 möglicherweise von anderen Entwicklern integriert werden als der Elektrochirurgie-Generator 12. Die Entwickler des Elektrochirurgie-Generators 12 können alle für den Elektrochirurgie-Generator 12 wichtigen spezifischen Parameterwertedaten und Steuerbefehle - gegebenenfalls in Abhängigkeit von dem in dem Generator-Datenspeicher 72 gespeicherten Betriebsprogramm 74 - als Betriebsvorgaben in dem Generator-Datenspeicher 72 ablegen. Solche Parameterwerte können beispielsweise maximal oder minimal zulässige Werte für die Ausgangsgleichspannung, die Ausgangswechselspannung, etc. sein.

Davon unabhängig, können Entwickler eines elektrochirurgischen Instruments 14 mithilfe der strukturierten Daten in dem Datensatz in dem Instrumenten-Datenspeicher 26 des elektrochirurgischen Geräts genau vorgeben, wie ein spezifischer Betriebsmodus für dieses elektrochirurgische Instrument 14 im Rahmen der durch das Betriebsprogramm 24 in dem Generator-Datenspeicher 72 ausgelesenen die Betriebsvorgaben 76 in dem Generator-Datenspeicher 72 ablaufen kann. Dabei müssen sich die Entwickler des elektrochirurgischen Instruments 14 keine weiteren Gedanken über das Betriebsprogramm 74 und die potentiell anwendbaren Betriebsvorgaben 76 machen. Vielmehr können die Entwickler des elektrochirurgischen Instruments 14 diese für den jeweiligen Elektrochirurgie-Generator 12 gegebene Betriebsvorgaben hinnehmen.

Während die strukturierten Daten in dem Nur-Lese-Bereich des Instrumenten-Datenspeichers 76 mittels eine Programmierschnittelle 82 beispielsweise bereits bei der Herstellung des elektrochirurgischen Instrumentes von einem Entwickler erzeugt werden, werden die in dem Schreib-Lese-Bereich des Instrumenten-Datenspeichers 26 gespeicherten Daten vorzugsweise nach jeder Nutzung des elektrochirurgischen Instruments 14 von dem Elektrochirurgie-Generator 12 entsprechende der Datenallokationstabelle in den Instrumenten-Datenspeicher 26 geschrieben und somit aktualisiert.

Den Entwicklern eines elektrochirurgischen Instruments 14 steht zum Definieren eines Betriebsmodus für das jeweilige elektrochirurgische Instrument 14 sowie zum Definieren der mit dem elektrochirurgischen Instrument 14 auszutauschenden und in dem Datenspeicher 26 des elektrochirurgischen Instruments 14 zu speichernden Daten eine Programmierschnittstelle 82 zur Verfügung.

Auf dem im Schreib-Lese-Bereich des elektrochirurgischen Instrumentes gespeicherten Zustands- und Nutzungsdaten können vom Elektrochirurgie-Generator beim Anschließen des elektrochirurgischen Instruments 14 an den Elektrochirurgie-Generator 12 oder während dessen Betriebs ausgelesen werden. Das Lesen der Zustands- und Nutzungsdaten erfolgt mithilfe der Datenallokationstabelle. Die Zustands- und Nutzungsdaten sind im Schreib-Lese-Bereich als Folge von Tag und Wert abgelegt (siehe Tabelle 3: Beispieldatensatz für gespeicherte Zustand- und Nutzungsdaten). Der Elektrochirurgie-Generator 12 liest also den Schreib-Lese-Bereich des Instrumenten-Datenspeichers 26 sequentiell aus und findet zunächst ein Tag (z. B. 0x01). Entsprechend der Datenallokationstabelle ist dem Elektrochirurgie-Generator 12 nun bekannt wie viele Daten folgen und um welche Daten es sich handelt (z. B. verbleibende Ultraschallaktivierungen mit einer Länge von 1 Byte). Anschließend findet der Elektrochirurgie-Generator 12 wieder ein Tag (z. B. 0x03). Entsprechend der Datenallokationstabelle ist dem Elektrochirurgie-Generator 12 nun bekannt wie viele Daten folgen und um welche Daten es sich handelt (z. B. abgegebene Aktivierungsenergie mit einer Länge von 2 Byte). Das wiederholt sich, bis der Generator das Ende-Tag 0x00 findet.

**Tabelle 3: Beispieldatensatz für gespeicherte Zustands- und Nutzungsdaten**

| **Adresse im Schreib-Lese-Bereich** | **Speicherinhalt** | **Beschreibung** |
|---|---|---|
| 0x0000 | 0x01 | 1. Tag |
| | | 0x01 = Nachfolgende Daten definieren die verbleibenden Ultraschallaktivierungen, die Länge der Daten beträgt 1 Byte (siehe Datenallokationstabelle) |
| 0x0001 | 0xff | 1, Daten |
| | | 0xff = 255 verbleibende Ultraschallaktivierungen |
| 0x0002 | 0x03 | 2. Tag |
| | | 0x03 = Nachfolgende Daten definieren die abgegebene Aktivierungsenergie, die Länge der Daten beträgt 2 Byte (siehe Datenallokationstabelle) |
| 0x0003 | 0xff | 2. Daten |
| 0x0004 | 0xff | 0xffff = 65535 mJ wurden bei der letzten Aktivierung abgegeben |
| 0x0005 | 0x00 | 3. Tag |
| | | 0x00 = Keine weitere Daten |

Welche Zustands- und Nutzungsdaten gespeichert werden sollen, wird in den strukturierten Daten in dem Instrumenten-Datenspeicher 26 des elektrochirurgischen Instruments 14 definiert. Zu diesem Zweck stehen spezifische, parametrisierbare Verweise zur Speicherung von Zustands- und Nutzungsdaten zur Verfügung. Ein Beispiel zum Schreiben der verbleibenden Ultraschallaktivierungen sowie der abgegebenen Aktivierungsenergie findet sich in Tabelle 4: Beispiel zur Definition von zu schreibenden Zustands- und Nutzungsdaten. Die Spalte "Resultierende Strukturierte Daten" wird in die den Betriebsmodus definierenden strukturierten Daten an entsprechender Stelle eingefügt und schließlich im Lese-Bereich des elektrochirurgischen Instrumentes abgelegt.

**Tabelle 4: Beispiel zur Definition von zu schreibenden Zustands- und Nutzungsdaten**

| **Verweis** | **Parameter 1** | **Parameter 2** | **Resultierende Strukturierte Daten** |
|---|---|---|---|
| **Write To Instrument** | **RTOU** | **255** | **0x4D 0x01 0xFF** |
| **Write To Instrument** | **Activation Energy** | **65535** | **0x4D 0x03 0xFF 0xFF** |

Die mit dem elektrochirurgischen Instrument 14 auszutauschenden und in dem Datenspeicher26 des elektrochirurgischen Instruments 14 zu speichernden Daten können somit über die Programmierschnittstelle 82 definiert (siehe Abbildung 1) und schließlich in dem Schreib-Lese-Bereich (Read-Write-Area) als Folge von *Bezeichner (Tag)* und *Wert* gespeichert werden. Ein Beispiel findet sich in der nachfolgenden Tabelle 5:

**Tabelle 5: Beispieldatensatz mit gespeicherten Nutzungs- und Zustandsdaten (kursiv)**

| **Partition** | **Daten** | **Beschreibung** |
|---|---|---|
| Read-write Area | *0x01* | *Tag 0 (0x01 = 1 Byte Zähler Ultraschallaktivierungen)* |
| | *0xff* | *Daten 0 (0xff = 255 Ultraschallaktivierungen übrig)* |
| | *0x03* | *Tag 1 (0x03 = 2 Byte Aktivierungsenergie )* |
| | *0xffff* | *Daten 1 (0xffff = 65535 mJ)* |
| | *0x00* | *Tag 2 (0x00 = 0 Byte Keine Daten)* |

Die Summe der Daten ergibt die Größe der Read-Write-Area und wird schließlich bei der Programmierung und Partitionierung des Datenspeichers 26 des elektrochirurgischen Instruments 14 in einem Konfigurationsbereich (Config-Area) abgespeichert. Der Elektrochirurgie-Generator 12 kann die Daten des Read-Write-Bereiches auslesen. Die Bezeichner (Tags) definieren, welche Daten der Elektrochirurgie-Generator 12 auslesen und verarbeiten soll und welche Daten er aktualisieren soll.

Über die Programmierschnittstelle 82 kann ein Entwickler die zwischen dem Elektrochirurgie-Generator 12 und dem elektrochirurgischen Instrument 14 auszutauschenden und zu speichernden Daten definieren. Ein Beispiel für eine Definition von Nutzungs- und Zustandsdaten mittels der Programmierschnittstelle 82 findet sich in Figur 6.

Über die Programmierschnittstelle 82 kann ein Entwickler außerdem einen Betriebsmodus für ein jeweiliges elektrochirurgisches Instrument 14 vollständig definieren. Dies schließt beispielsweise alle Strom- und Spannungsparameterwerte ein sowie auch Zeitvorgaben und Übergangsbedingungen für den Betrieb des elektrochirurgischen Instruments. Auf diese Weise kann die Entwicklung des Betriebsmodus mithilfe eines einfach zu bedienenden Werkzeugs nahezu ohne Software-Entwicklungskenntnisse durch einen Entwickler für ein elektrochirurgisches Instrument erfolgen. Die Programmierschnittstelle 82 stellt dem Entwickler eine Reihe von Parametersätzen zur Verfügung, mit denen dieser verschiedene Phasen, zum Beispiel die Anschnittsphase, die Schneidphase, die Koagulationsphase, aber auch Kurzschluss- oder Leistungsüberwachungen für das jeweilige elektrochirurgische Instrument definieren kann. Ein zu der Programmierschnittstelle 82 gehörendes Entwicklungswerkzeug erzeugt aus den Vorgaben einen speicherplatzsparenden Satz strukturierter Daten, der einen Datensatz bildet, der in dem Datenspeicher 26 des elektrochirurgischen Instruments 14 nichtflüchtig hinterlegt werden kann.

Falls ein durch strukturierte Daten in einem Datensatz 80 definierter, neuer Betriebsmodus für ein elektrochirurgisches Instrument auch eine Veränderung des Betriebsprogramms 74 in dem Generator-Datenspeicher 72 oder der Betriebsvorgaben 76 in dem Generator-Datenspeicher 72 erfordert, können derartige Veränderungen beispielsweise von einem mit dem Elektrochirurgie-Generator 12 vertrauten Entwickler über eine Programmierschnittstelle 82 vorgenommen werden. So kann sichergestellt werden, dass die einen Betriebsmodus definierten strukturierten Daten zu den Betriebsvorgaben 76 in dem Generator-Datenspeicher 72 und zu dem Betriebsprogramm 74 in dem Generator-Datenspeicher 72 kompatibel sind. Der Entwickler des elektrochirurgischen Instruments kann sich diesbezüglich mit dem mit dem Elektrochirurgie-Generator vertrauten Entwickler abstimmen. Im Ergebnis ist sichergestellt, dass ein nicht mit dem Elektrochirurgie-Generator vertrauter Entwickler - z.B. ein Entwickler für ein elektrochirurgisches Instrument - fehlerhafte Betriebsvorgaben erstellt oder das Betriebsprogramm so verändert, dass es zu unbeabsichtigten Effekten oder Fehlern kommt.

Wird ein elektrochirurgisches Instrument 14 an den Elektrochirurgie-Generator 12 angeschlossen, liest der Elektrochirurgie-Generator 12 den Instrumenten-Datenspeicher 26 in dem elektrochirurgischen Instrument 14 aus und interpretiert die strukturieren Daten in dem dort gespeicherten Datensatz 80 mit Hilfe der Betriebsvorgaben 76 in dem Generator-Datenspeicher 72, sodass die dort definierten Strom- und Spannungsverläufe inklusiver aller Zeitbedingungen und sonstiger Bedingungen angewandt werden. Auf diese Weise sind reduzierte Entwicklungszeiten und -kosten möglich. Das elektrochirurgische Instrument 14 kann weitestgehend unabhängig von einem Elektrochirurgie-Generator 12 integriert werden. Auch sind kürzere Produkteinführungszeiten möglich, da Betriebsmodi auch nach Einführen eines Elektrochirurgie-Generators 12 entwickelt und fertiggestellt werden können. Optimierungen eines Betriebsmodus und neuer Betriebsmodi können leicht durch aktualisierte oder neue elektrochirurgische Instrumente 14 eingeführt werden. Die Definition eines Betriebsmodus für ein elektrochirurgisches Instrument kann nahezu ohne Software-Entwicklungskenntnisse erfolgen.

Wenn das Elektrochirurgiesystem 10 mit einem an den Elektrochirurgie-Generator 12 angeschlossenen elektrochirurgischen Instrument 14 betrieben werden soll, steht der geeignete Betriebsmodus bereits nach Anschließen des elektrochirurgischen Instruments 14 zur Verfügung, weil die zugehörigen Datensätze 80 mit den strukturierten Daten von dem Instrumenten-Datenspeicher26 des elektrochirurgischen Instruments 14 ausgelesen werden können. Ein Nutzer muss daher nur noch beispielsweise einen Schalter 84 betätigen um das elektrochirurgische Instrument 14 in dem geeigneten Betriebsmodus des Elektrochirurgie-Generators 12 zu betreiben. Einstellungen oder Programmierungen muss der Nutzer nach Anschließen des elektrochirurgischen Instruments 14 nicht mehr vornehmen.

Der Schalter 84 ist über eine Leitung 86 mit dem Prozessor 70 des Elektrochirurgie-Generators 12 wirkverbunden, so dass mit Betätigen des Schalters 84 der Ablauf des in dem Generator-Datenspeicher 72 gespeicherten Betriebsprogramms 74 gestartet und unterbrochen werden kann. Der Schalter 84 kann ein Fußschalter sein, er kann aber auch ein Handschalter sein, der sich beispielsweise an dem elektrochirurgischen Instrument 14 befindet. Anstelle der Leitung 86 kann auch hier eine drahtlose Steuerverbindung vorgesehen sein.

Eine weitere Alternative zu einem Schalter 84 ist ein automatisches Starten des Betriebsprogramms, das ein Benutzer vorab aktivieren kann. In diesem Fall wird von dem elektrochirurgischen Instrument zunächst eine kleine Messspannung ausgegeben, um mit deren Hilfe einen Gewebekontakt (Stromfluss) zu detektieren. Wird ein Gewebekontakt - also ein Stromfluss - detektiert, wird das eigentliche Betriebsprogramm für das elektrochirurgische Instrument aufgerufen. Verschwindet der Gewebekontakt, wird das eigentliche Betriebsprogramm für das elektrochirurgische Instrument beendet, und es wird wieder eine kleine Messspannung ausgegeben, um mit deren Hilfe einen erneuten Gewebekontakt zu detektieren.

Durch das Betriebsprogram 74 gesteuert greift der Prozessor 70 während der Anwendung eines Betriebsmodus indirekt auf einzelne Betriebsvorgaben 76 in dem Generator-Datenspeicher 72 zu, indem zunächst ein Zugriff auf Verweise in der Datenstruktur 78 erfolgt und daraufhin ein Abruf derjenigen Betriebsvorgabe oder Betriebsvorgaben erfolgt, auf die ein jeweiliger Verweis verweist.

Abhängig sowohl von den Inhalten des Generator-Datenspeichers 76 als auch der Datenstruktur 78 sowie Signalen 90, die von den Erfassungseinheiten 54, 56 und 58 stammen, generiert der Prozessor 70 Steuersignale 88 für die Generatorsteuereinheit 46.

### Bezugszeichen

- 10: Elektrochirurgiesystem
- 12: Elektrochirurgie-Generator
- 14: elektrochirurgisches Instrument
- 16: Anschlusskabel
- 18.1, 18.2: elektrischen Ausgänge
- 18.3: Anschluss
- 20: Schaft
- 20.1, 20.2: Ausgänge
- 22: aktive Elektrode
- 24: Handgriff
- 26: Instrumenten-Datenspeicher
- 28, 30: Versorgungsleitungen
- 32: Datenleitung
- 40: Hochspannungsnetzteil
- 42: Ausgang
- 44: Hochfrequenzteil
- 46: Ausgangstransformator
- 48: Generatorsteuereinheit
- 50: Kondensator
- 52: Synchronisierschaltung
- 54: Ausgangsstromerfassungseinheit
- 56: Ausgangswechselspannungserfassungseinheit
- 58: Ausgangsgleichspannungserfassungseinheit
- 60: Hochspannungsgleichrichterschaltung
- 62: Ausgangskondensator
- 64: Schalter
- 70: Prozessor
- 72: Generator-Datenspeicher
- 74: Betriebsprogramm
- 76: Betriebsvorgaben
- 78: Datenstruktur
- 80: Datensatz
- 82: Programmierschnittstelle
- 84: Schalter
- 86: Leitung
- 88: Steuersignale des Prozessors
- 90: Signale der Erfassungseinheiten

## Patentansprüche

1. Elektrochirurgiesystem (10)
mit einem elektrochirurgischen Instrument (14) mit einem Instrumenten-Datenspeicher (26) und mit einem Anschluss für eine Anschlusskabel (16) oder einem angeschlossenen Anschlusskabel (16), das Versorgungsleitungen (28, 30) zum Versorgen der aktiven Elektrode (22) mit einem für eine elektrochirurgische Behandlung benötigten hochfrequenten Wechselstrom und wenigstens eine Datenleitung (32) zum Auslesen von Daten aus dem Instrumenten-Datenspeicher (26) sowie zum Schreiben von Daten in den Instrumenten-Datenspeicher (26) aufweist,
wobei der Instrumenten-Datenspeicher (26) zumindest einen Schreib-Lese-Bereich (Read-Write-Area) aufweist, auf der Zustands- und Nutzungsdaten unter Anwendung einer Datenallokationstabelle, die die Struktur des Schreib-Lese-Bereichs definiert, von einem Elektrochirurgie-Generator veränderbar abgelegt sind und
mit einem Elektrochirurgie-Generator (12), an den das elektrochirurgische Instrument (14) angeschlossen ist, wobei der Elektrochirurgie-Generator (12) über einen Prozessor (70) und mindestens einen Generator-Datenspeicher (72) verfügt, in dem
- erste Daten (74) gespeichert sind, die ein Betriebsprogramm zur Steuerung des Betriebs des Elektrochirurgie-Generators (12) in Verbindung mit dem elektrochirurgischen Instrument (14) definieren,
- Zweite Daten (76) gespeichert sind, die Betriebsvorgaben repräsentieren, die von dem Betriebsprogramm aufgerufen werden können und den Betrieb des Elektrochirurgie-Generator (12) beeinflussen können, aber keine festen Betriebsabläufe vorgeben,
- dritte Daten gespeichert sind, die eine Datenstruktur (78) mit Datensätze (80) mit strukturierten Daten gespeichert bilden, die Verweise auf in dem Generator-Datenspeicher (72) gespeicherte Betriebsvorgaben enthalten, die im Betrieb ein gezieltes Aufrufen einzelner Verweise durch das Betriebsprogramm erlauben, und
- vierte Daten gespeichert sind, die eine Datenallokationstabelle für einen Schreib-Lese-Bereich eines Instrumenten-Datenspeichers eines an den Elektrochirurgie-Generator (12) im Betrieb anzuschließenden elektrochirurgischen Instrumentes (14) bilden.

2. Elektrochirurgiesystem (10) gemäß Anspruch 1, bei dem die Datenstruktur (78) durch den Inhalt eines Nur-Lese-Bereichs des Instruenten-Datenspeichers (26) des elektrochirurgischen Instruments (14) gebildet ist.

3. Elektrochirurgiesystem (10) gemäß Anspruch 1 oder 2, bei dem der Prozessor (70) des Elektrochirurgie-Generators (12) in Verbindung mit dem Betriebsprogramm dazu konfiguriert ist, den Instrumeneten-Datenspeicher (26) des elektrochirurgischen Instruments auszulesen und/oder zu beschreiben.

4. Elektrochirurgiesystem (10) gemäß wenigstens einem der Ansprüche 1 bis 3, bei dem der Prozessor (70) des Elektrochirurgie-Generators (12) durch das Betriebsprogramm dazu konfiguriert ist, den Instrumenten-Datenspeicher (26) des elektrochirurgischen Instruments (14) während des Ablaufs des Betriebsprogramms auszulesen.

5. Elektrochirurgiesystem (10) gemäß wenigstens einem der Ansprüche 1 bis 4, mit einer Programmierschnittstelle (82) oder mehreren Programmierschnittstellen, mittels der die Inhalte insbesondere des Generator-Datenspeichers (72) und der Datenstruktur (78) programmiert werden können.

6. Elektrochirurgisches Instrument (14) für ein Elektrochirurgiesystem (10) gemäß den Ansprüchen 1 bis 5 wobei das elektrochirurgische Instrument (14) wenigstens eine aktive Elektrode (22) zum Bewirken einer elektrochirurgischen Behandlung, einen Instrumenten-Datenspeicher (26) und einen Anschluss für ein Anschlusskabel (16) oder ein angeschlossenes Anschlusskabel (16) aufweist, das Versorgungsleitungen (28, 30) zum Versorgen der aktiven Elektrode (22) mit einem für eine elektrochirurgische Behandlung benötigten hochfrequenten Wechselstrom und wenigstens eine Datenleitung (32) zum Auslesen von Daten aus dem Instrumenten-Datenspeicher (26) sowie zum Schreiben von Daten in den Instrumenten-Datenspeicher (26) aufweist,
wobei der Instrumenten-Datenspeicher (26) zumindest einen Schreib-Lese-Bereich (Read-Write-Area) aufweist, auf der Zustands- und Nutzungsdaten unter Anwendung einer Datenallokationstabelle, die die Struktur des Schreib-Lese-Bereichs definiert, von einem Elektrochirurgie-Generator veränderbar abgelegt sind.

7. Elektrochirurgisches Instrument (14) nach Anspruch 6, **dadurch gekennzeichnet, dass** der Instrumenten-Datenspeicher (26) partitioniert ist und eine einen Schreib-Lese-Bereich bildende erste Partition und einen Nur-Lese-Bereich bildende zweite Partition aufweist:

8. Elektrochirurgisches Instrument (14) nach Anspruch 6 oder 7 **dadurch gekennzeichnet, dass** der Instrumenten-Datenspeicher (26) Datensätze (80) mit strukturierten Daten enthält, die Verweise auf in einem Generator-Datenspeicher (72) eines Elektrochirurgie-Generators (12) gespeicherte Betriebsvorgaben (76) enthalten.

9. Elektrochirurgisches Instrument (14) nach den Ansprüchen 7 und 8, **dadurch gekennzeichnet, dass** die Datensätze (80) mit strukturierten Daten in der den Nur-Lese-Bereich bildenden Partition des Instrumenten-Datenspeichers (26) gespeichert sind.

10. Elektrochirurgisches Instrument (14) nach wenigstens einem der Ansprüche 6 bis 9, **dadurch gekennzeichnet, dass** der Instrumenten-Datenspeicher (26) des elektrochirurgischen Instruments (14) derart konfiguriert ist, dass ein Elektrochirurgie-Generator (12) im Betrieb des elektrochirurgischen Instruments (14) Nutzungs- und Zustandsdaten zu dem elektrochirurgischen Instrument (14) in die den Schreib-Lese-Bereich (Read-Write-Area) bildende Partition des Datenspeichers (26) schreiben kann.

11. Elektrochirurgisches Instrument (14) nach wenigstens einem der Ansprüche 6 bis 10, **dadurch gekennzeichnet, dass** der Instrumenten-Datenspeicher (26) ein nichtflüchtiger Speicher ist.

12. Elektrochirurgisches Instrument (14) nach wenigstens einem der Ansprüche 6 bis 11, **dadurch gekennzeichnet, dass** das elektrochirurgische Instrument (14) ein Handinstrument mit einem Handgriff (24) und einem Schaft (20) ist, an dem die aktive Elektrode (22) angeordnet ist.

13. Elektrochirurgisches Instrument (14) nach Anspruch 12, **dadurch gekennzeichnet, dass** der Instrumenten-Datenspeicher (26) in dem Handgriff (24) des elektrochirurgischen Instruments (14) untergebracht ist.

14. Elektrochirurgie-Generator (12) für ein Elektrochirurgiesystem (10) gemäß der Ansprüche 1 bis 5, mit Anschlüssen (8.1, 18.2, 18.3) zum Anschließen eines elektrochirurgischen Instruments (14) gemäß der Ansprüche 6 bis 13 sowie einem Prozessor (70) und mindestens einem Generator-Datenspeicher (72), der
- ein Betriebsprogramm zur Steuerung des Betriebs des Elektrochirurgie-Generators in Verbindung mit dem elektrochirurgischen Instrument (14) enthält,
- Betriebsvorgaben enthält, die von dem Betriebsprogramm aufgerufen werden können und den Betrieb des Elektrochirurgie-Generator (12) beeinflussen können, aber keine festen Betriebsabläufe vorgeben,
- eine Datenstruktur mit Datensätzen mit strukturierten Daten enthält, die Verweise auf in dem Generator-Datenspeicher (72) gespeicherte Betriebsvorgaben enthalten, und
- eine Datenallokationstabelle für einen Schreib-Lese-Bereich eines Instrumenten-Datenspeichers eines an den Elektrochirurgie-Generator (12) im Betrieb anzuschließenden elektrochirurgischen Instrumentes (14) enthält.
wobei der Prozessor (70) in Verbindung mit dem Betriebsprogramm dazu ausgebildet ist, im Betrieb des elektrochirurgischen Instruments (14) Nutzungs- und Zustandsdaten zu dem elektrochirurgischen Instrument (14) in die den Schreib-Lese-Bereich (Read-Write-Area) bildende Partition des Instrumenten-Datenspeichers (26) zu schreiben.

15. Verfahren zum Betreiben eines Elektrochirurgiesystems (10) gemäß einem der Ansprüche 1 bis 5, **dadurch gekennzeichnet, dass** der Elektrochirurgie-Generator (12) im Betrieb des elektrochirurgischen Instruments (14) Nutzungs- und Zustandsdaten zu dem elektrochirurgischen Instrument (14) in die den Schreib-Lese-Bereich (Read-Write-Area) bildende erste Partition des Datenspeichers (26) schreibt.

16. Verfahren nach Anspruch 15, bei dem Betriebsvorgaben und eine Datenstruktur (78) voneinander unabhängig bereitgestellt sind, wobei die Datenstruktur Verweise auf die Betriebsvorgaben enthält und ein Prozessor (70) des Elektrochirurgie-Generators (12) während des Ablaufs eines Betriebsprogramms indirekt auf einzelne Betriebsvorgaben zugreift, indem zunächst ein Zugriff auf Verweise in der Datenstruktur (78) erfolgt und daraufhin ein Abruf derjenigen Betriebsvorgabe oder Betriebsvorgaben erfolgt, auf die ein jeweiliger Verweis verweist und die Datenstruktur (78) aus einem Datenspeicher (26) eines elektrochirurgischen Instruments (14) ausgelesen wird.

17. Verfahren nach Anspruch 16, bei dem die Datenstruktur (78) und Zustands- und Nutzungsdaten nach Anschluss und vor einer Anwendung eines elektrochirurgischen Instruments (14) aus dem Datenspeicher (26) des elektrochirurgischen Instruments (14) ausgelesen und in der Datenstruktur auf dem elektrochirurgischen Instrument (14) enthaltene Datensätze (80) mit strukturierten Daten in eine Datenstruktur (78) übertragen werden, die auf einem Elektrochirurgie-Generator (12) gespeichert ist.
